# EUROPEAN PATENT APPLICATION

(11) **EP 4 201 516 A1**
(43) Date of publication of application: **28.06.2023**
(21) Application number: 21217611.9
(22) Date of filing: 23.12.2021
(51) Int. Cl.: B01J 23/34, B01J 23/889, C10G 2/00, B01J 21/06, B01J 37/18, B01J 35/00, B01J 37/02

(54) **MANGANESE TITANATE-CONTAINING FISCHER-TROPSCH CATALYST AND METHODS FOR MAKING AND USING SAME**

(71) Applicant: BP P.L.C., London SW1Y 4PD (GB)
(72) Inventor: The designation of the inventor has not yet been filed
(74) Representative: Hamer, Christopher K.

(57) **Abstract**

The present disclosure relates generally to titania-supported Fischer-Tropsch catalysts incorporating manganese titanate, methods of making and use thereof. In one aspect, the present disclosure provides a titania-supported Fischer-Tropsch catalyst precursor comprising a titania support, and disposed on the titania support, manganese in the range of 1 wt% to 20 wt%, calculated as manganese(0); wherein at least 10 at% of the manganese is in the form of MnTiO₃.

## Description

### BACKGROUND OF THE DISCLOSURE

### FIELD

The present disclosure relates to Fischer-Tropsch catalysts containing manganese titanate, processes for preparation thereof, and uses thereof.

### TECHNICAL BACKGROUND

The conversion of synthesis gas (i.e., a mixture of carbon monoxide and hydrogen, also known as syngas) into hydrocarbons by the Fischer-Tropsch process has been known for decades, but has historically lagged in performance compared to other hydrocarbon synthesis techniques. The growing importance of alternative energy sources has resulted in renewed interest in the Fischer-Tropsch (FT) process as it allows a direct and environmentally acceptable route to high-quality fuels and feedstock chemicals.

FT processes are known for producing linear hydrocarbons for use in fuels, as well as oxygenates which can be useful in fuels and also serve as valuable feedstock chemicals. The hydrocarbon fuel derived from FT processes is typically better able to meet increasingly stringent environmental regulations compared to conventional refinery-produced fuels, as FT-derived fuels typically have lower contents of sulfur, nitrogen, and aromatic compounds, which contribute to the emission of potent pollutants such as SO₂, NOₓ, and particulates. Alcohols and other oxygenates obtained may also be used as reagents in other processes, such as in the synthesis of lubricants.

A variety of transition metals have been identified to be catalytically active in the conversion of synthesis gas into hydrocarbons and oxygenated derivatives thereof. In particular, cobalt, nickel, and iron have been studied, often in combination with a support material, of which the most common are alumina, silica and carbon.

In the typical preparation of supported cobalt-containing FT synthesis catalysts, a solid support material is contacted with a solution of a soluble cobalt compound, such as cobalt nitrate. The impregnated support is subsequently calcined and/or oxidized to form a cobalt oxide, typically one or more of CoO, Co₂O₃, or Co₃O₄, sometimes in combination with oxides of one or more promoters. Such oxides typically have poor FT catalytic activity and must be reduced to form the preferred catalytically-active cobalt metal species. The nature of the cobalt metal formed in combination with optional promoters can have large effects on the catalyst activity and selectivity. Further, certain catalyst compositions are known to sinter and degrade over time, impairing catalyst performance.

Accordingly, there exists a need to develop improved Fischer-Tropsch catalysts and methods of making them.

### SUMMARY

The present inventors have developed catalyst compositions and processes for making thereof that allow high manganese loading with low oxygenate selectivity. This results in increased catalyst stability and activity while maintaining control over the Fischer-Tropsch reaction selectivity, for example, to provide a desired high proportion of paraffinic hydrocarbons and a low proportion of oxygenates such as alcohols. Advantageously, the catalyst precursor may be prepared *ex situ* and then, in certain embodiments, followed by a subsequent cobalt impregnation step to generate the Fischer-Tropsch catalyst.

Thus, in one aspect, the disclosure provides for a titania-supported Fischer-Tropsch catalyst precursor comprising manganese in the range of 1 wt% to 50 wt%, calculated as manganese(0); wherein at least 10 at% of the manganese is in the form of manganese titanate (i.e., MnTiO₃) as measured by x-ray diffraction and preferably at least 25%, more preferably at least 50% and most preferably at least 75% of manganese is present in the form of MnTiO₃. Any unconverted manganese species would likely be forms of manganese oxide such as MnO, Mn₂O₃, Mn₃O₄, MnO₂, but could also be present in the form of MnTi₂O₄ or Mn₂TiO₄ spinels.

In another aspect, the present disclosure provides for a process for the production of a titania-supported Fischer-Tropsch catalyst support, for example the catalyst support precursor as otherwise described herein, the process comprising:
providing a first support precursor comprising manganese salt disposed on a titania support, wherein the first catalyst precursor comprises no cobalt; and optionally calcining in an oxygen containing atmosphere;
contacting the support precursor with a reducing gas at a temperature of at least 350 °C, for a time sufficient to provide a titania-supported Fischer Tropsch catalyst precursor in which at least 10 at% of manganese is present as MnTiO₃.

Other aspects of the disclosure will be apparent to those skilled in the art to use alternative techniques to X-ray diffraction, such as beamline X-ray adsorption techniques (EXAFS/XANES) or beamline X-ray diffraction as a more accurate method for observing and quantifying the phases formed.

In another aspect, the present disclosure provides for the production of a titania-supported Fischer-Tropsch catalyst precursor, the process comprising:
a) providing a first catalyst support precursor comprising a manganese salt disposed on a titania support, wherein the first catalyst precursor comprises no cobalt; and optionally calcining the first catalyst support precursor in an oxygen containing atmosphere;
b) contacting the first catalyst support precursor with a reducing gas at a temperature of at least 350 °C, for a time sufficient to provide a titania-supported Fischer Tropsch catalyst precursor in which at least 10 at% of manganese is present as MnTiO₃
c) contacting the catalyst precursor with a decomposable cobalt salt andcalcining at a temperature in the range of 200 °C to 450 °C in an oxygen containing atmosphere;
d) reducing the catalyst precursor with a reducing gas at a temperature of no more than 350 °C to form the titania-supported Fischer-Tropsch catalyst wherein the final catalyst has at least 10 at% of manganese is present as MnTiO₃.

In another aspect, the present disclosure provides for a process for the production of a Fischer-Tropsch catalyst, the process comprising:
providing the Fischer-Tropsch catalyst precursor as otherwise described herein;
impregnating the catalyst precursor with a decomposable cobalt salt;
calcining the impregnated catalyst precursor at a temperature in the range of 200 °C to 450 °C.

In another aspect, the present disclosure provides for a process for the production of hydrocarbons, the process comprising contacting the manganese titanate-containing supported Fischer-Tropsch catalyst containing at least 1 wt% cobalt and 1 wt% mangangese, for example, the catalyst as otherwise described herein, or prepared by a process as otherwise described herein, with a gaseous reaction mixture comprising H₂ and CO in a ratio in the range of 1:1 to 3:1, wherein the process has a selectivity for alcohols of no more than 10% (e.g., no more than 8%, or no more than 6%, or no more than 5%).

In another aspect, the present disclosure provides for a method for controlling the selectivity of a Fischer-Tropsch catalyst, the method comprising:
(i) providing a titania-supported Fischer-Tropsch synthesis catalyst precursor comprising manganese in an amount of at least 1 wt%, as calculated as manganese(0); and
(ii) contacting the catalyst with a reducing gas at a temperature of at least 350 °C for a time sufficient to provide a second catalyst precursor in which at least 10 at% of manganese is present as MnTiO₃;
(iii) impregnating the second catalyst precursor with a cobalt salt;
(iv) calcining at a temperature in the range of 200 °C to 450 °C;
(v) contacting the calcined catalyst with a reducing gas at a temperature of no more than 350 °C (e.g., no more than 300 °C) to produce the Fischer-Tropsch catalyst, wherein
the Fischer-Tropsch catalyst has a selectivity for alcohols that is no more than 50% of a selectivity for alcohols of an equivalent catalyst contacted with a reducing gas in step (ii) at a temperature of 300 °C for the same amount of time. For example, comparing catalyst performances under FT conditions such as 30barg and H₂:CO ratio of 2:1 where either temperature or flowrate are adjusted to match conversions to 40-60%.

Other aspects of the disclosure will be apparent to those skilled in the art in view of the description that follows.

### BRIEF DESCRIPTION OF THE DRAWINGS

Fig. 1 is a schematic depiction of a reactor suitable for use in the processes described herein, wherein the reduction gas is fed (101) over the catalyst bed (120) to carry out the transformation to MnTiO₃. Optionally, the exit gas (104) can be recycled back into the feed (103).
Fig. 2 showing the X-ray diffraction patterns of 3 catalyst support examples where MnTiO₃ is formed
Fig. 3 shows the X-ray diffraction pattern of the support material calcined in air versus the treatment with hydrogen
Fig. 4 shows the X-ray diffraction of the reduced support, and the reduced support impregnated with cobalt and calcined, showing the loss of crystal structure of the MnTiO₃ phase, and the air calcined analogues.

### DETAILED DESCRIPTION

The present disclosure in various aspects relates to Fischer-Tropsch catalyst compositions comprising manganese titanate. Typically, the inclusion of promoters has the effect of altering catalyst selectivity and activity. For example, the presence of manganese (in the form of MnOₓ) in typical cobalt-containing Fischer-Tropsch catalysts has the effect of increasing selectivity for oxygenates, such as alcohols, over hydrocarbons. The present inventors have noted, however, that the inclusion of manganese in Fischer-Tropsch catalyst can provide several advantageous effects, including decreased thermal sintering and catalyst damage, increased catalyst life stability, increased chain growth capability, and low methane selectivity. Accordingly, the inventors have sought a method to decouple these technical effects, for example to provide low oxygenate selectivity while maintaining increased catalyst stability.

Surprisingly, the present inventors have found that conversion of at least a portion of manganese deposited on a titania support into manganese titanate yields a catalyst material with reduced oxygenate selectivity but maintaining increased catalyst stability. The support bearing manganese titanate can be prepared *ex situ* and these properties can be substantially maintained throughout the catalyst life. In various embodiments as otherwise described herein, this conversion is achieved through the high-temperature treatment of a manganese species deposited on a titania support, resulting in at least partial conversion of the manganese species into manganese titanate (i.e., MnTiO₃). Without wishing to be bound by theory, it is presently believed that the manganese titanate contributes advantageous structural properties while being largely inert to Fischer-Tropsch synthesis reactions. Accordingly, the disclosed catalysts and method of making thereof surprisingly allow for the capture of certain beneficial qualities of manganese inclusion while maintaining the ability to tune catalyst selectivity towards paraffinic products.

Additionally, the manganese titanate phase formed as a result of the hydrogen reduction at greater than 350 °C has been surprisingly found to be reversible. Further, it has been identified that and calcination in air can be used to at least partially decompose the MnTiO₃ phase back to manganese oxides. These processes advantageously allow precise tuning of MnTiO₃ and manganese oxide content, which leads to tuning of catalyst properties. Accordingly, in some embodiments, the process as otherwise described herein further comprises calcining the MnTiO₃-containing support, catalyst precursor, or catalyst in air. For example, the calcining may be so as to convert at least a portion of the MnTiO₃ to MnO (e.g., at least 10 at%, or at least 20 at%).

Advantageously, catalysts prepared as described herein may be made in two steps, wherein the formation of manganese titanate is done separately from cobalt impregnation. This allows *ex situ* manganese titanate formation. As the formation of manganese titanate requires temperatures of at least 350 °C, the manganese transformation can be effected in a separate, specialized reactor. Subsequent to cobalt impregnation, the calcining and final reduction may be performed at lower temperatures in a typical reactor that lacks the specialized metallurgy for high temperature operation. This allows for decreased capital costs in reactor construction.

Of particular interest is the re-reduction of the catalyst with cobalt present, wherein the reduction process can be completed at below 350 °C such that the cobalt oxides are reduced and the manganese titanate phase is reformed. Thereby avoiding the need to reach high temperatures which were required for the original MnTiO₃ phase formation.

Accordingly, in one aspect, the disclosure provides for a titania-supported Fischer-Tropsch catalyst precursor comprising a titania support; and disposed on the titania support, manganese in the range of 1 wt% to 50 wt%, calculated as manganese(0); wherein at least 10 at% of the manganese is in the form of manganese titanate (i.e., MnTiO₃) as measured by x-ray diffraction.

The titania-supported Fischer-Tropsch catalyst precursor is formed before cobalt addition. Accordingly, in various embodiments as otherwise described herein, the catalyst precursor comprises no cobalt. For example, in certain embodiments, the catalyst precursor comprises no cobalt, copper, or iron. In particle embodiments, the catalyst precursor contains no other transition metals beyond titanium and manganese.

The percentage of manganese that is in the form of manganese titanate may be tuned by the skilled person in light of the present disclosure. Without intending to be bound by theory, it is believed that increasing the proportion of manganese in the form of manganese titanate will reduce the oxygenate selectivity of the catalyst. For example, in various embodiments as otherwise described herein, at least 15 at% (e.g., at least 20 at%, or at least 30 at%, or at least 40 at%) of the manganese is in the form of MnTiO₃ as measured by X-ray diffraction In various embodiments as otherwise described herein, at least 50 at% (e.g., at least 60 at%, or at least 70 at%, or at least 80 at%) of the manganese is in the form of MnTiO₃ as measured by X-ray diffraction. In various embodiments as otherwise described herein, at least 90 at% (e.g., at least 95 at%, or at least 98 at%) of the manganese is in the form of MnTiO₃ as measured by x-ray diffraction. The person of ordinary skill in the art, can, based on the disclosure herein, provide reduction conditions (e.g., via increased time and/or temperature) to provide a desired proportion of manganese titanate.

In some embodiments, however, it may be advantageous to provide a portion of the manganese not in the form of the manganese titanate. For example, a person of ordinary skill in the art may select synthesis conditions to maintain a portion of manganese as a catalytically active species, for example MnO. Accordingly, in some embodiments as otherwise described herein, no more than 99 at% (e.g., no more than 95 at%, or no more than 90 at%, or no more than 85 at%, or no more than 80 at%) of the manganese is in the form of MnTiO₃ as measured by X-ray diffraction. In various embodiments as otherwise described herein, less than 5 at% of the manganese is in the form of MnO as measured by X-ray diffraction. For example, in certain embodiments, less than 3 at% or less than 2 at%, or less than 1.5 at%) of the manganese is in the form of MnO as measured by X-ray diffraction or suitable X-ray technique.

The catalyst compositions and catalyst precursor compositions described herein comprise manganese in various loadings as selected by the person of ordinary skill in the art. The manganese loading is determined as the total of manganese present in all manganese-bearing species, including, for example, manganese titanate, manganese oxide, and manganese metal. In various embodiment as otherwise described herein, manganese is present in an amount of at least 0.5 wt%. For example, in particular embodiment, manganese is present in an amount of at least 1 wt% (e.g., at least 1.5 wt%, or at least 2 wt%). Advantageously, the present invention allows for relatively high manganese loadings while controlling the effect of manganese on the Fischer-Tropsch synthesis product selectivity. Accordingly, the skilled person would recognize that an upper limit to the manganese loading is not practical. For example, in some embodiments, manganese is present in the catalyst composition or catalyst precursor in an amount of no more than 50 wt%, e.g., no more than 40 wt%, or no more than 30 wt%. In various embodiments as otherwise described herein, manganese is present in an amount in the range of 1 wt% to 15 wt%, for example, 1-10 wt%, or 1-8 wt%, or 1-5 wt%. In various embodiments as otherwise described herein, manganese is present in an amount in the range of 2 to 50 wt%, e.g., 2-30 wt%, or 2-20 wt%, or 2-15 wt%. In various embodiments as otherwise described herein, manganese is present in an amount in the range of 4 to 30 wt%, e.g., 4-20 wt% or 4-15 wt%. In various embodiments as otherwise described herein, manganese is present in an amount in the range of 5 to 25 wt%, e.g., 5-15 wt% or 5-12 wt% or 5-10 wt%. Manganese loadings are calculated as manganese(0).

Advantageously, the manganese (in whatever form they may be, e.g., metallic, oxide, or mixed oxide) is disposed on a titania support. A titania support includes at least 50 wt% titania. In various embodiments as otherwise described herein, the titania support comprises at least 80 wt% titania, for example or at least 85 wt%, or at least 90 wt% titania. In various embodiments, the titania support comprises at least 95 wt% titania, e.g., at least 98 wt% titania. In some embodiments the titania support is substantially all titania (e.g., titania but for negligible amounts of impurities. In various particular embodiments, the titania support comprises no more than 5 wt% (e.g., no more than 1 wt%, or no more than 0.5 wt%) total of silica, alumina, ceria, zirconia, and zinc oxide. The titania support can be provided in a variety of forms, e.g., as an extrudate, powder or microspheres form.

Without wishing to be bound by theory, it is presently believed that at least a portion of the manganese titanate forms a solid solution with the titania support. Accordingly, when the composition of the support is discussed herein, the proportions are understood to be exclusive of any manganese titanate species.

No particular arrangement is implied by the term "disposed on." The inventors contemplate that the cobalt and the manganese can be "disposed on" the titania support in a variety of fashions, e.g., as would be achieved through impregnation (such as incipient wetness techniques), precipitation, or coextrusion techniques.

In another aspect, the present disclosure provides for a process for the production of a titania-supported Fischer-Tropsch catalyst precursor, for example the catalyst as otherwise described herein, the process comprising:
providing a support precursor comprising manganese salt disposed on a titania support;
contacting the support precursor with a reducing gas at a temperature of at least 350 °C, for a time sufficient to provide a titania-supported Fischer Tropsch catalyst precursor in which at least 10 at% of the manganese is present as MnTiO₃.

The present inventors have determined that high-temperature reduction processes can produce a catalyst support suitable for subsequent impregnation with a cobalt. The resulting catalyst has a relatively high selectivity for paraffinic hydrocarbons and a low selectivity for oxygenates such as alcohols. Thus, the catalyst can enjoy the structural benefits of the presence of manganese, without the sometimes undesirable production of alcohols. The present inventors have correlated this with the formation of a manganese titanate phase (MnTiO₃).

Thus, the contacting the first catalyst precursor with a reducing gas may be performed at a variety of temperatures of at least for a time sufficient to provide a desired degree of manganese titanate. In various embodiments as otherwise described herein, the contacting with a reducing gas is performed for a time of at least 10 minutes, e.g., at least 30 minutes, or at least an hour. In various embodiments, the contacting with a reducing gas is performed for a time in the range of 10 minutes to 24 hours, e.g., 30 minutes to 24 hours, or 1-24 hours.

The inventors have found that high temperatures are critical to the production of the manganese titanate phase. Accordingly, as noted above, the reduction temperature is at least 350 °C. However, higher temperatures can advantageously be used. For example, in various embodiments as otherwise described herein, the contacting of the first catalyst precursor with the reducing gas is performed at a temperature of at least 360 °C, for example, at least 370 °C, or at least 380 °C, or at least 390 °C. In various embodiments as otherwise described herein, the contacting of the first catalyst precursor with the reducing gas is performed at a temperature of at least 400 °C, e.g., at least 410 °C, or at least 420 °C. In various embodiments as otherwise described herein, the contacting the first catalyst precursor with a reducing gas is performed at a temperature of at least 430 °C, e.g., at least 440 °C, or at least 450 °C. In various embodiments, the contacting of the first catalyst precursor with the reducing gas is performed at a temperature of no more than 800 °C, for example, no more than 700 °C. In various embodiments, the contacting of the first catalyst precursor with the reducing gas is performed at a temperature of no more or no more than 650 °C,

The high-temperature reduction as described herein is operating at a temperature above that of most other Fischer-Tropsch processes. Reaching such temperatures usually requires specialized reactors with upgraded metallurgy in order to reduce the chance of reactor damage. Accordingly, it may be desirable to conduct the high-temperature reduction in a reaction specialized for that purpose. Accordingly, in certain embodiments as otherwise described herein, the high-temperature reduction occurs in a first reactor. Subsequently, in some embodiments, the Fischer-Tropsch catalyst precursor may be removed from the first reactor and introduced into a second reactor for subsequent treatment steps.

In various embodiments as otherwise described herein, the reducing gas comprises H₂, CO or mixtures thereof. For example, in particular embodiments, H₂ and/or CO are present in the reducing gas in the range of 10 vol% to 100 vol%. For example, in certain embodiments, H₂ is present in the reducing gas in the range of 20 vol% to 100 vol%, or 30 vol% to 100 vol%, or 40 vol% to 100 vol%, or 50 vol% to 100 vol%. In various embodiments, the reducing gas may further comprise an inert gas such as N₂ or CO₂ in an amount of no more than 90 vol%, for example, no more than 80 vol%, or 70 vol%, or 60 vol%. In various embodiments, the reducing gas is a mixture of H₂ and N₂.

The contacting of the first catalyst precursor with the reducing gas may be performed at variety of pressures, for example, in the range of 0 to 40 barg. In certain embodiments, use of a low pressure may be desirable. In various such embodiments, the contacting the first catalyst precursor with a reducing gas may be performed at pressures in the range of 0 to 15 barg, for example 0 to 10 barg, or 0 to 5 barg, or 0 to 2 barg.

As described above, the person of skill in the art will select a desirable relative amount of manganese titanate for the catalyst. The relative amount of manganese that is in the form of manganese titanate may be tuned by the skilled person in light of the present disclosure by varying the conditions of the contacting step, for example, to provide any of the relative amounts described above. Typically, higher temperature, longer time and higher partial pressure of reductant (e.g., hydrogen) will provide a greater conversion of manganese salts to manganese titanate. In various embodiments as otherwise described herein, at least 10 at% (e.g., at least 15 at%, or at least 20 at%, or at least 30 at%, or at least 40 at%) of the manganese of the manganese salt of the first catalyst precursor is converted to manganese titanate, as measured by X-ray diffraction.

In various embodiments, the high temperature reduction may be carried out so that a majority of the manganese of the manganese oxide is converted into manganese titanate. Accordingly, in various embodiments as otherwise described herein, at least 50 at% (e.g., at least 60 at%, or at least 70 at%, or at least 80 at%) of the manganese of the manganese oxide of the first catalyst precursor is converted to manganese titanate, as measured by X-ray diffraction. In various embodiments as otherwise described herein, at least at least 90 at% (e.g., at least 95 at%, or at least 98 at%) of the manganese of the manganese oxide of the first catalyst precursor is converted to manganese titanate, as measured by X-ray diffraction.

However, as described above, in some cases it may be desirable to leave a fraction of the manganese in the form of manganese oxide. Thus, in some embodiments, at least 1 at% of the manganese of the manganese oxide (e.g., at least 0.5 at%, or at least 1 at%, or at least 2 at%, or at least 5 at%) remains in the form of manganese oxide after the high-temperature reduction.

The first catalyst precursor that is subjected to the high temperature reduction can be provided by the person of ordinary skill in the art. Numerous methods of production of supported transition metal catalysts are known in the art. For example, impregnation methods are described in International Patent Application nos. PCT/EP2015/080745 and PCT/EP2018/053350, each of which is hereby incorporated herein by reference in its entirety.

The reduction temperature subsequent to cobalt addition may be adjusted by the skilled person. Typically, it is desired that at least a portion of cobalt is reduced to cobalt(0) as the catalytically active Fischer-Tropsch species. Accordingly, reducing the catalyst precursor with a reducing gas may be performed at a temperature of no more than 340 °C, e.g., no more than 330 °C, or 320 °C, or 310 °C, or 300 °C. In various embodiments, the reduction is performed at a temperature of at least 200 °C.

The catalyst compositions described herein may comprise cobalt in a various loadings as selected by the person of ordinary skill in the art. In various embodiments as otherwise described herein, cobalt is present in an amount in the range of 5 wt% to 25 wt%, (e.g., in the range of 7-35 wt%, or 10-35 wt%, or 5-25 wt%, or 7-25 wt%, or 10-25 wt%, or 5-20 wt%, or 7-20 wt%, or 10-20 wt%, calculated as cobalt(0)).

It is known to be beneficial to perform Fischer-Tropsch catalysis with a shaped particle, such as an extrudate, particularly in the case of fixed catalyst bed reactor systems. For instance, it is known that, for a given shape of catalyst particles, a reduction in the size of the catalyst particles in a fixed bed gives rise to a corresponding increase in pressure drop through the bed. Thus, the relatively large shaped particles cause less of a pressure drop through the catalyst bed in the reactor compared to the corresponding powdered or granulated supported catalyst. Shaped particles, such as extrudates, also generally have greater strength and experience less attrition, which is of particular value in fixed bed arrangements where bulk crush strength must be very high. Accordingly, in certain embodiments as otherwise descried herein, the titania support is the form of an extrudate.

Reference herein to "impregnation" or "impregnating" is intended to refer to contact of the support material with a solution, or solutions, of, for example, a cobalt-containing compound or a manganese-containing compound, as required, before drying in order to achieve precipitation of the cobalt-containing compound and/or the manganese-containing compound. Impregnation with a substantially dissolved solution, or solutions, of a cobalt-containing compound and/or a manganese-containing compound ensures good dispersion of the cobalt-containing compound and the manganese-containing compound on the support material and is thus preferred. This is in contrast, for instance, to the use of partially dissolved cobalt-containing compound and/or a partially dissolved manganese-containing compound in 'solid solutions' or suspensions, where the level of dispersion of the cobalt-containing compound and manganese-containing compound across the surface, and in the pores, of the support material can fluctuate depending on the nature of the precipitation on the support material. Furthermore, use of a fully dissolved solution, or solutions, of cobalt-containing compound and/or manganese-containing compound also has less of an impact upon the resulting morphology and bulk crush strength of an extrudate formed thereafter compared with solid solutions. Nevertheless, benefits of the processes of the present disclosure can also be realized in the case where a solid solution, or solutions, of a partially undissolved cobalt-containing compound and/or manganese-containing compound is used.

Where a powder or granulate of a support material is contacted with a solution, or solutions, of cobalt-containing compound and/or manganese-containing compound, the amount of solution used preferably corresponds to an amount of liquid which is suitable for achieving a mixture which is of a suitable consistency for further processing, for example for shaping by extrusion. In that case, complete removal of the solvent of the impregnating solution may be effected after formation of the shaped particle, such as an extrudate.

Suitable cobalt-containing compounds are those which are thermally decomposable to an oxide of cobalt following calcination and which are preferably completely soluble in the impregnating solution. Preferred cobalt-containing compounds are the nitrate, acetate or acetylacetonate of cobalt, most preferably the nitrate of cobalt, for example cobalt nitrate hexahydrate. It is preferred to avoid the use of the halides because these have been found to be detrimental.

Suitable manganese-containing compounds are those which are thermally decomposable following calcination and which are preferably completely soluble in the impregnating solution. Preferred manganese-containing compounds are the nitrate, acetate or acetylacetonate of manganese, most preferably the acetate of manganese.

The solvent of the impregnating solution(s) may be either an aqueous solvent or a non-aqueous, organic solvent. Suitable non-aqueous organic solvents include, for example, alcohols (e.g. methanol, ethanol and/or propanol), ketones (e.g. acetone), liquid paraffinic hydrocarbons and ethers. Alternatively, aqueous organic solvents, for example an aqueous alcoholic solvent, may be employed. Preferably, the solvent of the impregnating solution(s) is an aqueous solvent.

Impregnation of the support material is usually followed by drying of the impregnating solution in order to effect precipitation of the cobalt-containing compound and/or the manganese-containing compound on to the support material and preferably also to remove bound solvent of the impregnating solution (e.g. water). Drying therefore does not, for instance, lead to full decomposition of the cobalt- or manganese containing compound or otherwise lead to a change in oxidation state of the cobalt- or manganese containing compound. As will be appreciated, in embodiments where an extrusion is performed, complete drying and removal of solvent (e.g. bound solvent) of the impregnating solution may occur after forming of a shaped particle, for example by extrusion. Drying is suitably conducted at temperatures from 50 °C to 150 °C, preferably 75 °C to 125 °C. Suitable drying times are, for example, from 5 minutes to 72 hours. Drying may suitably be conducted in a drying oven or in a box furnace, for example, under the flow of an inert gas at elevated temperature.

The supported Co-Mn Fischer-Tropsch synthesis catalyst used in the process of the present disclosure may additionally comprise one or more promoters, dispersion aids or binders. Promoters may be added to promote reduction of an oxide of cobalt to cobalt metal, preferably at lower temperatures. Preferably, the one or more promoters is selected from the list consisting of ruthenium, palladium, platinum, rhodium, rhenium, chromium, nickel, iron, molybdenum, manganese, tungsten, zirconium, gallium, thorium, lanthanum, cerium, copper and mixtures thereof. Promoter is typically used in a cobalt to promoter atomic ratio of up to 250:1 and more preferably up to 125:1, still more preferably up to 25:1, and most preferably 10:1. In one or more preferred embodiments, the one or more promoters are present in the cobalt- containing Fischer-Tropsch synthesis catalyst obtained in an amount from 0.1 wt% to 3 wt%, on an elemental basis, based on the total weight of the supported synthesis catalyst. In embodiments where manganese is added as a promoter, this is understood to be manganese in addition to that added during impregnation. In one or more preferred embodiments, the cobalt-containing Fischer-Tropsch synthesis catalyst does not contain one or more promoters. For example, in some embodiments as otherwise described herein, the cobalt-containing Fischer-Tropsch synthesis catalyst comprises transition metals, wherein the transition metals consist of Co, Mn, and Ti.

In certain embodiments as otherwise described herein, the catalyst support precursor is prepared through a process comprising:
impregnating a titania support material with a decomposable manganese salt to form an impregnated titania support material; and
calcining the impregnated titania support material at a temperature in the range of 200 °C to 450 °C.

Advantageously, the presently disclosed catalysts and processes for making thereof allow the production of catalysts with high manganese loading combined with desirably low selectivity for alcohols when utilized in a Fischer-Tropsch synthesis process. Accordingly, in another aspect, the present disclosure provides for a process for the production of hydrocarbons, the process comprising contacting a titania-supported Fischer-Tropsch catalyst, for example, the catalyst as otherwise described herein, or prepared by a process as otherwise described herein, the process comprising contacting a titania-supported Fischer-Tropsch catalyst containing at least 1 wt% cobalt and at least 1 wt% manganese, for example, the catalyst as otherwise described herein, or prepared by a process as otherwise described herein, with a gaseous reaction mixture comprising H₂ and CO in a ratio in the range of 1:1 to 3:1, wherein the process has a selectivity for alcohols of no more than 10%. For example, in various embodiments as otherwise described herein, the process has a selectivity for alcohols of no more than 8%, or no more than 6%, or even no more than 5%. In various such embodiments, the catalyst includes at least 2 wt% manganese, e.g., at least 4 wt% or even at least 7 wt% or even at least 10wt% or even at least 15wt% manganese.

The Fischer-Tropsch process as otherwise described herein may also have an advantageously high C₅₊ selectivity. For example, in certain embodiments, the process has a C₅₊ selectivity of at least 75%, for example, at least 80%, or at least 85%, or at least 90%. The Fischer-Tropsch process as otherwise described herein may also have an advantageously low methane selectivity. For example, in certain embodiments, the process has a methane selectivity of no more than 6%, for example, no more than 5%, or 4%, or 3%.

Conventional Fischer-Tropsch temperatures may be used in order to prepare liquid hydrocarbons in accordance with the present disclosure, using the catalysts described herein. For example, the temperature of the contacting of a mixture of hydrogen and the gaseous carbon oxide (e.g., in the form of a synthesis gas mixture) with a supported cobalt-manganese Fischer-Tropsch catalyst may suitably be in the range from 100 to 400 °C, such as from 100 to 350 °C, or 100 to 300 °C, or 100 to 250 °C, or 150 to 400 °C, or 150 to 350 °C, or 150 to 300 °C, or 150 to 250 °C. In certain embodiments, the contacting is conducted at a temperature of no more than 350 °C, e.g., no more than 325 °C, or no more than 300 °C, or no more than 280 °C, or no more than 260 °C. The pressure of the contacting (i.e., the temperature of the Fischer-Tropsch reaction) can in certain embodiments suitably be in the range from 10 to 100 bara (from 1 to 10 MPa), such as from 15 to 75 bara (from 1.5 to 7.5 MPa), or from 20 to 50 bara (from 2.0 to 5.0 MPa). For example, in certain embodiments the contacting is conducted at a pressure of no more than 7.5 MPa absolute.

In particular embodiments, the temperature of the Fischer-Tropsch reaction is in the range from 150 to 350 °C, more preferably from 180 to 300 °C, and most preferably from 200 to 260 °C. In preferred embodiments, the pressure of the Fischer-Tropsch reaction is in the range from 10 to 100 bar (from 1 to 10 MPa), more preferably from 10 to 60 bar (from 1 to 6 MPa) and most preferably from 20 to 45 bar (from 2 to 4.5 MPa).

As discussed, the present disclosure provides methods for controlling the selectivity of a Fischer-Tropsch synthesis catalyst. Accordingly, in another aspect, the present disclosure provides for a method for controlling the selectivity of a Fischer-Tropsch catalyst, the method comprising:
(i) providing a titania-supported Fischer-Tropsch synthesis catalyst precursor comprising manganese in an amount of at least 1 wt%, as calculated as manganese(0) (e.g., the catalyst precursor as otherwise described herein); and
(ii) contacting the catalyst with a reducing gas at a temperature of at least 350 °C for a time sufficient to provide a second catalyst precursor in which at least 10 at% of manganese is present as MnTiO₃;
(iii) impregnating the second catalyst precursor with a cobalt salt;
(iv) calcining at a temperature in the range of 200 °C to 450 °C;
(v) contacting the calcined catalyst with a reducing gas at a temperature of no more than 300 °C to produce the Fischer-Tropsch catalyst, wherein
the Fischer-Tropsch catalyst has a selectivity for alcohols that is no more than 50% of a selectivity for alcohols of an equivalent catalyst contacted with a reducing gas in step (ii) at a temperature of 300 °C for the same amount of time.

For example, in certain embodiments as otherwise described herein, the alcohol selectivity of the resulting reduced catalyst is no more than 35% (e.g., no more than 20%) of the alcohol selectivity of the equivalent catalyst reduced at a temperature 300 °C. Beyond alcohol selectivity, the C₅₊ hydrocarbon selectivity may also be increased by the methods as otherwise described herein. Accordingly, in certain embodiments as otherwise described herein, the resulting reduced catalysts has a C₅₊ selectivity greater than the equivalent catalyst (e.g., at least 110% of the C₅₊ selectivity of the equivalent catalyst) reduced at 300 °C. In this aspect, selectivities may be determined under FT condition, for example, comparing catalyst performances under FT conditions such as 30barg and H2:CO ratio of 2:1 where either temperature or flowrate are adjusted to match conversions to 40-60%.

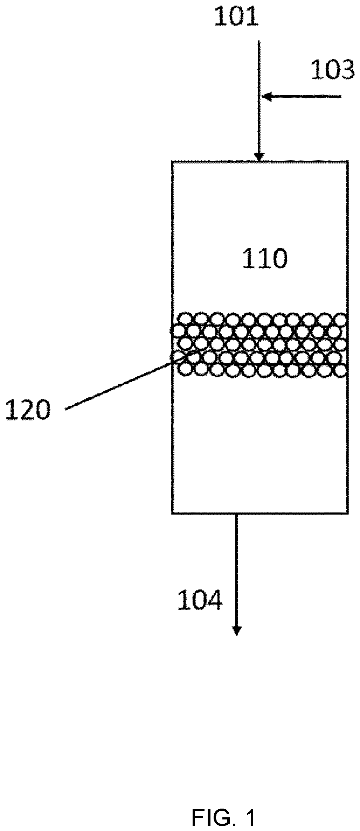

As described in more detail below, a variety of reaction systems can be used in performing the processes described herein, and the person of ordinary skill in the art will adapt conventional systems as necessary. An example of a reaction system for use in performing the processes of the disclosure is shown in schematic view in FIG. 1. Reactor 110 is charged with reduced catalyst 120. Syngas is introduced through inlet 101, and hydrocarbon products are removed through outlet 104 for further processing. Unreacted syngas, and optionally light hydrocarbons, may be removed through a suitable recycle outlet, and recycled back into inlet 101 through recycle feed 103.

CO conversion is defined as moles of CO used/moles of CO fed x 100. C₅₊ hydrocarbon selectivity is defined as moles of CO attributed to C₅₊ hydrocarbons/moles of CO converted x 100. Alcohol selectivity is defined as moles of CO attributed to alcohols/moles of CO converted x 100.

Unless otherwise stated, temperatures referred to in the Examples are applied temperatures and not catalyst/bed temperatures. Unless otherwise stated, pressures referred to in the Examples are absolute pressures.

The processes of the disclosure will now be further described by reference to the following Examples which are illustrative only.

In particular embodiments, the terms cobalt salt or manganese salt, the term 'salt' includes but not limited to nitrates, acetates, hydroxides, oxides, carbonates, citrates, chlorides and sulphates.

### EXAMPLES

The Examples that follow are illustrative of specific embodiments of the methods of the disclosure, and various uses thereof. They are set forth for explanatory purposes only, and are not to be taken as limiting the scope of the disclosure.

### Example 1: Manganese loading and reduction temperature screening

Three titania-supported Fischer-Tropsch catalyst precursors were prepared with metal loadings 50%, 30%, and 10% MnO by weight. These catalyst precursors were prepared by mixing titania (P25) with a decomposable manganese salt such as manganese acetate tetrahydrate or manganese nitrate and calcining at a temperature of at least 450 °C to form MnO. Subsequently, they were reduced in H₂ at atmospheric pressure at 500 °C for 12 h:

**Table 1**

| **Material** | **Wt% TiO₂** | **Wt% MnO** | **Wt% Co** | **Calcination** |
|---|---|---|---|---|
| Support 1 | 50 | 50 | 0 | H₂ to 500 °C |
| Support 2 | 70 | 30 | 0 | |
| Support 3 | 90 | 10 | 0 | |

The above supports were then impregnated with a cobalt salt (cobalt nitrate hexahydrate) to form the following catalysts:

**Table 2**

| **Catalyst** | **Support** | **Wt% TiO₂** | **Wt% MnO** | **Wt% Co** | **Calcination** |
|---|---|---|---|---|---|
| Catalyst 4 | Support 1 | 45 | 45 | 10 | Air at 300 °C |
| Catalyst 5 | Support 2 | 63 | 27 | 10 | |
| Catalyst 6 | Support 3 | 81 | 9 | 10 | |

Subsequently, Fischer-Tropsch syntheses were performed with Catalyst 6, above, as compared to a Co-Mn catalyst that had not undergone high temperature reduction, and a manganese-free cobalt catalyst. Fischer-Tropsch synthesis was performed at 30 barg, 225 °C with a 4300 hr⁻¹ syngas space velocity and a syngas ratio of 1.8 H₂:CO:

**Table 3**

| **Catalyst description** | **CO Conv. %** | **CH₄ sel. %** | **C₅₊ sel. %** | **C₂₋₄ sel. %** | **C₁-C₈ alcohols** | **C₂-C₈ olefin** | **Temp.** | **Prep** |
|---|---|---|---|---|---|---|---|---|
| Catalyst 6 (10%Co/9%Mn/TiO₂) | 45.3 | 5.6 | 88.3 | 6.1 | 1.4 | 8.8 | 225 °C | High T/H2 calcined support |
| Catalyst 7: Comparative preparation (10%Co/10%Mn/TiO₂) | 19.2 | 6.3 | 65.7 | 28.1 | 17.3 | 30.0 | 225 °C | Normal preparation without high T/H₂ |
| Catalyst 8: Comparative example (10%Co/TiO₂) | 37.4 | 7.3 | 86.1 | 6.6 | 2.3 | 9.4 | 225 °C | Normal prep. (no Mn) |

As observed above, inventive Catalyst 6 showed minor alcohol selectivity of only 1.4 %, compared to 17.3% observed for the equivalent catalyst that did not undergo high temperature reduction. Interestingly, the alcohol selectivity for Catalyst 6 was even less than the selectivity of 2.3% observed for Catalyst 8, which contained no manganese. Accordingly, high temperature reduction in this way is an effective route to alter alcohol and oxygenate selectivity.

### Example 2: Formation of Manganese Titanate

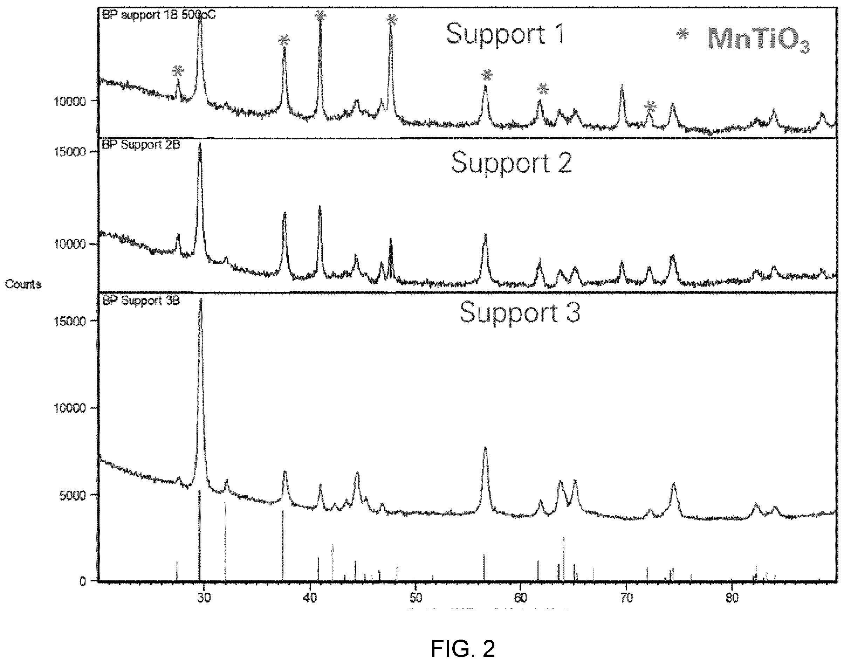

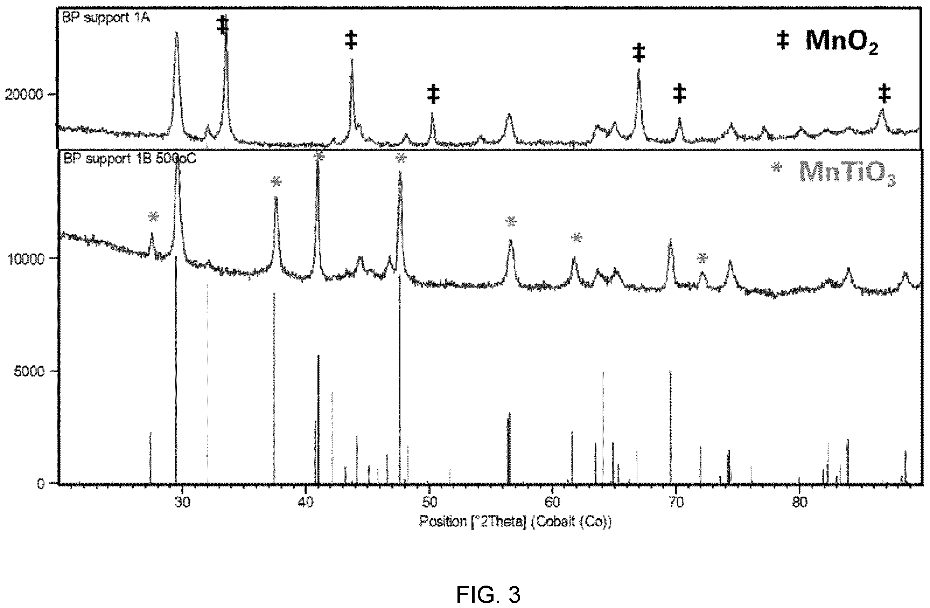

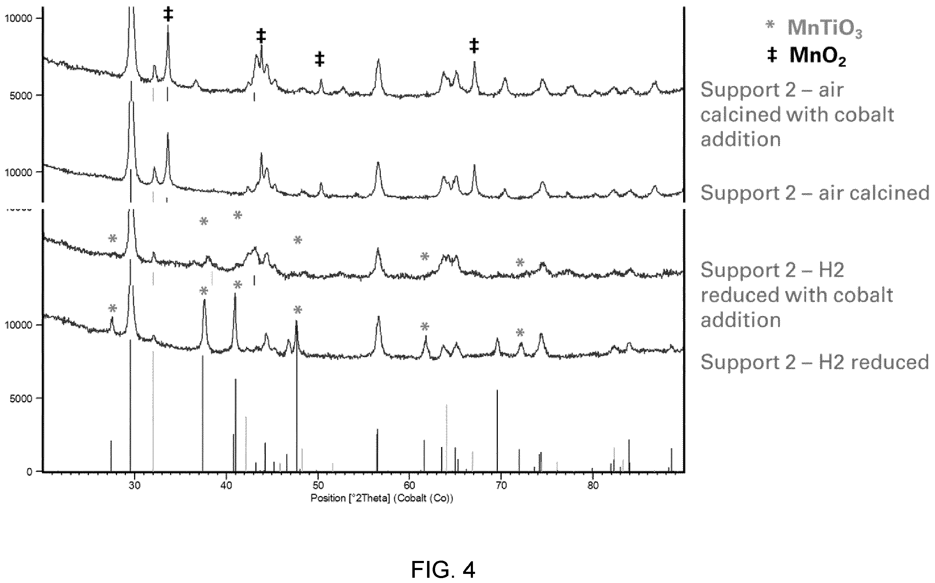

Figure 2 shows the X-ray diffraction analysis of supports 1, 2 and 3. In particular all three supports formed the MnTiO₃ phase with 50% of the manganese present as MnTiO₃ as indicated in table 4. Figure 3 shows the impact of calcination in air versus hydrogen, Fig. 4 shows the X-ray diffraction of the reduced support, and the reduced support impregnated with cobalt and calcined, showing the loss of crystal structure of the MnTiO₃ phase, and the air calcined analogues. In situ X-ray diffraction showed that the lost MnTiO₃ crystal structure could be regained at temperatures below 350 °C.

**Table 4**

| Sample | wt% TiO₂ | wt% MnO | wt% Co(m) | Wt% MnTiO₃ | Wt% MnO | Wt% MnO₂ |
|---|---|---|---|---|---|---|
| Support 1 | 50 | 50 | 0 | 37 | 26 | - |
| Support 2 | 70 | 30 | 0 | 38 | 11 | - |
| Support 3 | 90 | 10 | 0 | 22 | 0 | - |
| Support 1 (calcined in air) | 50 | 50 | 0 | - | - | 46 |
| Support 2 (calcined in air) | 70 | 30 | 0 | - | - | 25 |
| Support 3 (calcined in air) | 90 | 10 | 0 | - | - | 10 |

These catalysts with 10%Cobalt, 0-10%Manganese on titania were reduced and analysed by X-ray absorption spectroscopy (XAS, EXAFS, XANES) to demonstrate the formation of MnTiO₃ and metallic cobalt. Table 5 and Table 6 show the formation of Mn-Mn and Mn-Ti bonds in line with the titanate phase, while cobalt metal and cobalt oxide were the only cobalt phases observed. Further, the average coordination number (CN) is observed to depart significantly from that expected for manganese oxides and approach the lower coordination numbers expected for titanate phases.

**Table 5 EXAFS fitting results at the Mn edge for 10%Co-5%Mn/TiO₂**

| Reduction in H₂ | **Mn-Mn/ Å** | **CN** | **Mn-Ti/ Å** | **CN** |
|---|---|---|---|---|
| 300 °C | 3.12 | 4.8 | - | - |
| 500 °C | 3.07 | 3.2 | 3.47 | 2.1 |
| 550 °C | 3.06 | 3.9 | 3.41 | 2.5 |
| Reference MnO | 3.14 | 12 | - | - |
| Reference Mn₂O₃ | 3.13 | 6 | - | - |
| Reference MnO₂ | 3.418 | 8 | - | - |
| Reference MnTiO₃ | 3.063 | 3 | 3.433 | 3 |
| Reference MnTi₂O₄ | 3.724 | 4 | - | - |

**Table 6 EXAFS fitting results at the Mn edge for 10%Co-10%Mn/TiO₂**

| Reduction in H₂ | **Mn-Mn/ Å** | **CN** | **Mn-Ti/ Å** | **CN** |
|---|---|---|---|---|
| 300 °C | 3.12 | 7.3 | - | - |
| 500 °C | 3.07 | 4.0 | 3.43 | 2.3 |
| 550 °C | 3.05 | 4.5 | 3.43 | 3.0 |
| Reference MnO | 3.14 | 12 | - | - |
| Reference Mn₂O₃ | 3.13 | 6 | - | - |
| Reference MnO₂ | 3.418 | 8 | - | - |
| Reference MnTiO₃ | 3.063 | 3 | 3.433 | 3 |
| Reference MnTi₂O₄ | 3.724 | 4 | - | - |

Various exemplary embodiments of the disclosure include but are not limited to the enumerated embodiments of the claims as listed below, which can be combined in any number and in any combination that is not technically or logically inconsistent.

Embodiment 1. A titania-supported Fischer-Tropsch catalyst precursor comprising:
a titania support, and disposed on the titania support,
manganese in the range of 1 wt% to 50 wt%, calculated as manganese(0);
wherein at least 10 at% of the manganese is in the form of MnTiO₃ to form a catalyst support.

Embodiment 2. The catalyst precursor of embodiment 1, wherein the catalyst precursor comprises no cobalt (e.g., comprises no cobalt, copper, or iron).

Embodiment 3. The catalyst precursor of embodiment 1 or embodiment 2, wherein at least 15 at% (e.g., at least 20 at%, or at least 30 at%, or at least 40 at%) of the manganese is in the form of MnTiO₃

Embodiment 4. The catalyst precursor of embodiment 1 or embodiment 2, wherein at least 50 at% (e.g., at least 60 at%, or at least 70 at%, or at least 80 at%, at least 90 at%, at least 95 at%, or at least 98 at%)) of the manganese is in the form of MnTiO₃.

Embodiment 5. The catalyst precursor of any of embodiments 1-4, wherein at least 0.1 at% of the manganese is in the form of MnO (e.g., at least 0.5 at%, or at least 1 at%, or at least 2 at%, or at least 5 at%).

Embodiment 6. The catalyst precursor of any of embodiments 1-5, wherein manganese is present in an amount in the range of 1 wt% to 50 wt%, for example, 1-30 wt%, or 1-20 wt%, or 1-15 wt%. 2 wt% to 40 wt%, 2-20 wt%, or for example 4 wt% to 35 wt%, for example, 4-30 wt%, or 4-20 wt%, 7 wt% to 25 wt%, for example, 7-20 wt%.

Embodiment 7. The catalyst precursor of any of embodiments 1-6, wherein the titania support comprises at least 80 wt% titania, e.g., at least 85 wt% or at least 90 wt% titania, at least 95 wt% titania, e.g., at least 98 wt% titania or at least 99 wt% titania.

Embodiment 8. The catalyst precursor of any of embodiments 1-7, wherein the support material comprises no more than 5 wt% (e.g., no more than 1 wt%, or no more than 0.5 wt%) total of silica, alumina, ceria, zirconia, and zinc oxide.

Embodiment 9. The catalyst precursor of any of embodiments 1-8, wherein the support material is in the form of an extrudate, powder, granular or microsphere.

Embodiment 10. The catalyst precursor of any of embodiments 1-9, wherein the catalyst precursor is made via an impregnation technique, chemical deposition, or spray drying.

Embodiment 11. A process for the production of a titania-supported Fischer-Tropsch catalyst precursor (e.g., the catalyst precursor of any of embodiments 1-10), the process comprising:
providing a first catalyst precursor comprising a manganese oxide disposed on a titania support, wherein the support precursor comprises no cobalt; and
contacting the first catalyst precursor with a reducing gas at a temperature of at least 350 °C, for a time sufficient to provide a second catalyst precursor in which at least 10 at% of manganese is present as MnTiO₃.

Embodiment 12. The process of embodiment 11, the contacting with the reducing gas is performed for a time in the range of 10 minutes to 24 hours, e.g., 30 minutes to 24 hours, or 1-24 hours.

Embodiment 13. The process of claim any of embodiments 11-12, wherein the reducing gas comprises H₂, CO or a mixture thereof, wherein the reducing gas is present in the range of 10 vol% to 100 vol% of the reducing gas.

Embodiment 14. The process of any of embodiments 1-13, wherein the contacting with a reducing gas is performed at a temperature of at least 360 °C (e.g., at least 370 °C, or at least 380 °C, or at least 390 °C at least 400 °C (e.g., at least 410 °C, or at least 420 °C at least 440 °C, or at least 450 °C).

Embodiment 15. The process of any of embodiments 11-14, wherein the contacting with the reducing gas is performed at a temperature of no more than 800 °C, e.g., no more than 700 °C or no more than 600 °C.

Embodiment 16. The process of any of claims 11-15, wherein the contacting with a reducing gas is performed at a pressure in the range of 0 barg to 40 barg.

Embodiment 17. The process of any of embodiments 11-16, wherein at least 10 at% (e.g., at least 15 at%, or at least 20 at%, or at least 30 at%, or at least 40 at%) of the manganese in the support precursor is converted to manganese titanate.

Embodiment 18. The process of any of embodiments 11-17, wherein at least 50 at% (e.g., at least 60 at%, or at least 70 at%, or at least 80 at% or at least 90 at%, or at least 95 at%) of the manganese in the support precursor is converted to manganese titanate.

Embodiment 19. The process of any of embodiments 11-18, wherein the first catalyst precursor is prepared through a process comprising:
impregnating a titania support material with a decomposable manganese salt; and
calcining the impregnated titania support material at a temperature in the range of 200 °C to 450 °C.

Embodiment 20. A process for the production of a Fischer-Tropsch catalyst, the process comprising:
providing the Fischer-Tropsch catalyst precursor of any of claims 1-10 (or prepared according to any of claims 11-19);
impregnating the catalyst precursor with a decomposable cobalt salt;
calcining the impregnated catalyst precursor at a temperature in the range of 200 °C to 450 °C.

Embodiment 21. The process of embodiment 20, wherein cobalt is present in the produced catalyst in an amount in the range of 5 wt% to 25 wt%, (e.g., in the range of 7-35 wt%, or 10-35 wt%, or 5-25 wt%, or 7-25 wt%, or 10-25 wt%, or 5-20 wt%, or 7-20 wt%, or 10-20 wt%, calculated as cobalt(0)).

Embodiment 22. A Fischer-Tropsch catalyst prepared by the process of embodiment 20 or embodiment 21.

Embodiment 23. A process for the production of hydrocarbons, comprising contacting a MnTiO₃ containing supported Fischer-Tropsch catalyst containing at least 1 wt% cobalt and 1 wt% manganese (e.g., the catalyst of embodiment 22, or made according to the process of embodiments 20 or 21) with a gaseous reaction mixture comprising H₂ and CO in a ratio in the range of 1:1 to 3:1, wherein the process has a selectivity for alcohols of no more than 20% (e.g., no more than 10%, or no more than 8%, or no more than 6%).

Embodiment 24. The process of embodiment 23, wherein the catalyst includes at least 2 wt% manganese, e.g., at least 4 wt% or even at least 7 wt%.

Embodiment 25. The process of embodiment 23 or embodiment 24, wherein the process has a C₅₊ selectivity of at least 75% (e.g., at least 80%, or at least 85%, or at least 90%).

Embodiment 26. The process of any of embodiments 23-25, wherein the process has a methane selectivity of no more than 15% (e.g., no more than 10%, or 8%)

Embodiment 27. A method for controlling the selectivity of a Fischer-Tropsch catalyst, the method comprising:
(i) providing a titania-supported Fischer-Tropsch synthesis catalyst precursor comprising manganese in an amount of at least 1 wt%, as calculated as manganese(0); and
(ii) contacting the catalyst with a reducing gas at a temperature of at least 350 °C for a time sufficient to provide a second catalyst precursor in which at least 10 at% of manganese is present as MnTiO₃;
(iii) impregnating the second catalyst precursor with a cobalt salt;
(iv) calcining at a temperature in the range of 200 °C to 450 °C;
(v) contacting the calcined catalyst with a reducing gas at a temperature of no more than 300 °C to produce the Fischer-Tropsch catalyst; wherein
the Fischer-Tropsch catalyst has a selectivity for alcohols that is no more than 50% of a selectivity for alcohols of an equivalent catalyst contacted with a reducing gas in step (ii) at a temperature of 300 °C for the same amount of time.

Embodiment 28. The method of embodiment 27, wherein the alcohol selectivity of the resulting reduced catalyst is no more than 35% of the alcohol selectivity of the equivalent catalyst (e.g., no more than 20%).

Embodiment 29. The method of embodiment 27 or embodiment 28, wherein the resulting Fischer-Tropsch catalyst has a C₅₊ selectivity greater than the equivalent catalyst (e.g., at least 110% of the C₅₊ selectivity of the equivalent catalyst).

Embodiment 30. A method for producing a supported Fischer-Tropsch catalyst comprising:
a) providing a first catalyst support precursor comprising manganese salt disposed on a titania support, wherein the first catalyst precursor comprises no cobalt; and optionally calcining in an oxygen-containing atmosphere to form a support precursor
b) contacting the first catalyst support precursor with a reducing gas at a temperature of at least 350 °C, for a time sufficient to provide a titania-supported Fischer Tropsch catalyst precursor in which at least 10 at% of manganese is present as MnTiO₃;
c) contacting the catalyst precursor with a decomposable cobalt salt and calcining at a temperature in the range of 200 °C to 450 °C in an oxygen containing atmosphere; wherein the MnTiO₃ phase is at least partially decomposed;
d) reducing the catalyst precursor with a reducing gas at a temperature of no more than 350 °C to form the titania-supported Fischer-Tropsch catalyst wherein the final catalyst has at least 10 at% of manganese is present as MnTiO₃.

Embodiment 31. The catalyst precursor of embodiment 30, wherein step b is carried out at greater than 350 °C preferably greater than 400 °C more preferably greater than 450 °C and most preferably greater than 500 °C.

Embodiment 32. The catalyst precursor of embodiment 30 or embodiment 31, wherein step d is carried out at less than 350 °C preferably less than 320 °C more preferably less than 300 °C and most preferably greater than 280 °C.

The particulars shown herein are by way of example and for purposes of illustrative discussion of certain embodiments of the present disclosure only and are presented in the cause of providing what is believed to be the most useful and readily understood description of the principles and conceptual aspects of various embodiments of the disclosure. In this regard, no attempt is made to show details associated with the methods of the disclosure in more detail than is necessary for the fundamental understanding of the methods described herein, the description taken with the examples making apparent to those skilled in the art how the several forms of the methods of the disclosure may be embodied in practice. Thus, before the disclosed processes and devices are described, it is to be understood that the aspects described herein are not limited to specific embodiments, apparatus, or configurations, and as such can, of course, vary. It is also to be understood that the terminology used herein is for the purpose of describing particular aspects only and, unless specifically defined herein, is not intended to be limiting.

The terms "a," "an," "the" and similar referents used in the context of describing the methods of the disclosure (especially in the context of the following embodiments and claims) are to be construed to cover both the singular and the plural, unless otherwise indicated herein or clearly contradicted by context.

All methods described herein can be performed in any suitable order of steps unless otherwise indicated herein or otherwise clearly contradicted by context. The use of any and all examples, or exemplary language (e.g., "such as") provided herein is intended merely to better illuminate the methods of the disclosure and does not pose a limitation on the scope of the disclosure. No language in the specification should be construed as indicating any non-claimed element essential to the practice of the methods of the disclosure.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to". Words using the singular or plural number also include the plural and singular number, respectively. Additionally, the words "herein," "above," and "below" and words of similar import, when used in this application, shall refer to this application as a whole and not to any particular portions of the application.

As will be understood by one of ordinary skill in the art, each embodiment disclosed herein can comprise, consist essentially of or consist of its particular stated element, step, ingredient or component. As used herein, the transition term "comprise" or "comprises" means includes, but is not limited to, and allows for the inclusion of unspecified elements, steps, ingredients, or components, even in major amounts. The transitional phrase "consisting of" excludes any element, step, ingredient or component not specified. The transition phrase "consisting essentially of' limits the scope of the embodiment to the specified elements, steps, ingredients or components and to those that do not materially affect the embodiment.

All percentages, ratios and proportions herein are by weight, unless otherwise specified.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

Groupings of alternative elements or embodiments of the disclosure are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Some embodiments of various aspects of the disclosure are described herein, including the best mode known to the inventors for carrying out the methods described herein. Of course, variations on these described embodiments will become apparent to those of ordinary skill in the art upon reading the foregoing description. The skilled artisan will employ such variations as appropriate, and as such the methods of the disclosure can be practiced otherwise than specifically described herein. Accordingly, the scope of the disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

The phrase "at least a portion" as used herein is used to signify that, at least, a fractional amount is required, up to the entire possible amount.

In closing, it is to be understood that the various embodiments herein are illustrative of the methods of the disclosures. Other modifications that may be employed are within the scope of the disclosure. Thus, by way of example, but not of limitation, alternative configurations of the methods may be utilized in accordance with the teachings herein. Accordingly, the methods of the present disclosure are not limited to that precisely as shown and described.

## Claims

1. A titania-supported Fischer-Tropsch catalyst precursor comprising:
a titania support, and disposed on the titania support, manganese in the range of 1 wt% to 50 wt%, calculated as manganese(0);
wherein at least 10 at% of the manganese is in the form of MnTiO₃ to form a catalyst support.

2. The catalyst precursor of claim 1, wherein the catalyst precursor comprises no cobalt (e.g., comprises no cobalt, copper, or iron).

3. The catalyst precursor of claim 1 or claim 2, wherein at least 15 at% (e.g., at least 20 at%, or at least 30 at%, or at least 40 at%) of the manganese is in the form of MnTiO₃

4. The catalyst precursor of claim 1 or claim 2, wherein at least 50 at% (e.g., at least 60 at%, or at least 70 at%, or at least 80 at%, at least 90 at%, at least 95 at%, or at least 98 at%)) of the manganese is in the form of MnTiO₃.

5. The catalyst precursor of any of claims 1-4, wherein at least 0.1 at% of the manganese is in the form of MnO (e.g., at least 0.5 at%, or at least 1 at%, or at least 2 at%, or at least 5 at%).

6. The catalyst precursor of any of claims 1-5, wherein manganese is present in an amount in the range of 1 wt% to 50 wt%, for example, 1-30 wt%, or 1-20 wt%, or 1-15 wt%. 2 wt% to 40 wt%, 2-20 wt%, or for example 4 wt% to 35 wt%, for example, 4-30 wt%, or 4-20 wt%, 7 wt% to 25 wt%, for example, 7-20 wt%.

7. The catalyst precursor of any of claims 1-6, wherein the titania support comprises at least 80 wt% titania, e.g., at least 85 wt% or at least 90 wt% titania, at least 95 wt% titania, e.g., at least 98 wt% titania or at least 99 wt% titania.

8. A process for the production of a titania-supported Fischer-Tropsch catalyst precursor (e.g., the catalyst precursor of any of claims 1-7), the process comprising:
providing a first catalyst precursor comprising a manganese oxide disposed on a titania support, wherein the support precursor comprises no cobalt; and
contacting the first catalyst precursor with a reducing gas at a temperature of at least 350 °C, for a time sufficient to provide a second catalyst precursor in which at least 10 at% of manganese is present as MnTiO₃.

9. A process for the production of a Fischer-Tropsch catalyst, the process comprising:
providing the Fischer-Tropsch catalyst precursor of any of claims 1-7 (or prepared according to claim 8);
impregnating the catalyst precursor with a decomposable cobalt salt;
calcining the impregnated catalyst precursor at a temperature in the range of 200 °C to 450 °C.

10. The process of claim 9, wherein cobalt is present in the produced catalyst in an amount in the range of 5 wt% to 25 wt%, (e.g., in the range of 7-35 wt%, or 10-35 wt%, or 5-25 wt%, or 7-25 wt%, or 10-25 wt%, or 5-20 wt%, or 7-20 wt%, or 10-20 wt%, calculated as cobalt(0)).

11. A Fischer-Tropsch catalyst prepared by the process of claim 9 or claim 10.

12. A process for the production of hydrocarbons, comprising contacting a MnTiO₃ containing supported Fischer-Tropsch catalyst containing at least 1 wt% cobalt and 1 wt% manganese (e.g., the catalyst of claim 11, or prepared according to claim 9 or claim 10) with a gaseous reaction mixture comprising H₂ and CO in a ratio in the range of 1:1 to 3:1, wherein the process has a selectivity for alcohols of no more than 20% (e.g., no more than 10%, or no more than 8%, or no more than 6%).

13. A method for controlling the selectivity of a Fischer-Tropsch catalyst, the method comprising:
(i) providing a titania-supported Fischer-Tropsch synthesis catalyst precursor comprising manganese in an amount of at least 1 wt%, as calculated as manganese(0); and
(ii) contacting the catalyst with a reducing gas at a temperature of at least 350 °C for a time sufficient to provide a second catalyst precursor in which at least 10 at% of manganese is present as MnTiO₃;
(iii) impregnating the second catalyst precursor with a cobalt salt;
(iv) calcining at a temperature in the range of 200 °C to 450 °C;
(v) contacting the calcined catalyst with a reducing gas at a temperature of no more than 300 °C to produce the Fischer-Tropsch catalyst, wherein
the Fischer-Tropsch catalyst has a selectivity for alcohols that is no more than 50% of a selectivity for alcohols of an equivalent catalyst contacted with a reducing gas in step (ii) at a temperature of 300 °C for the same amount of time.

14. The method of claim 13, wherein the alcohol selectivity of the resulting reduced catalyst is no more than 35% of the alcohol selectivity of the equivalent catalyst (e.g., no more than 20%).

15. The method of claim 13 or claim 14, wherein the resulting Fischer-Tropsch catalyst has a C₅₊ selectivity greater than the equivalent catalyst (e.g., at least 110% of the C₅₊ selectivity of the equivalent catalyst).

16. A method for producing a supported Fischer-Tropsch catalyst, the method comprising:
a) providing a first catalyst support precursor comprising manganese salt disposed on a titania support, wherein the first catalyst precursor comprises no cobalt; and optionally calcining in an oxygen containing atmosphere to form a support precursor
b) contacting the first catalyst support precursor with a reducing gas at a temperature of at least 350 °C, for a time sufficient to provide a titania-supported Fischer Tropsch catalyst precursor in which at least 10 at% of manganese is present as MnTiO₃;
c) contacting the catalyst precursor with a decomposable cobalt salt and calcining at a temperature in the range of 200 °C to 450 °C in an oxygen containing atmosphere, wherein the MnTiO₃ phase is at least partially decomposed
d) reducing the catalyst precursor with a reducing gas at a temperature of no more than 350 °C to form the titania-supported Fischer-Tropsch catalyst wherein the final catalyst has at least 10 at% of manganese is present as MnTiO₃.

17. The catalyst precursor of claim 15 or claim 16, wherein step d is carried out at less than 350 °C preferably less than 320 °C more preferably less than 300 °C and most preferably greater than 280 °C.
